# EUROPEAN PATENT APPLICATION

(11) **EP 4 710 957 A1**
(43) Date of publication of application: **18.03.2026**
(21) Application number: 25000079.1
(22) Date of filing: 29.07.2025
(51) Int. Cl.: A61L 27/26, A61L 27/38, A61L 27/48, A61L 27/54

(54) **SCAFFOLD WITH STEM CELLS**

(30) Priority: 13.09.2024 IT 202400020416
(71) Applicant: Parri, Umberto, 56025 San Miniato (PI) (IT)
(72) Inventor: Parri, Umberto, 56025 San Miniato (PI) (IT)

(57) **Abstract**

Scaffold with stem cells comprises 60 per cent biomaterial, consisting of 75 per cent Silicone by weight, Poli Glycolic Acid at 1.87 per cent, Xanthan Gum at 1.25 per cent, NaCl at 3.75 per cent, Agar at 3.75 per cent, Carbon Fullerene C60 at 4.38 per cent and water at 2.5 per cent. All reacted with 7.5 per cent Silicone catalyst and a 40 per cent predominantly cellular materials composed of ASC, i.e. mesenchymal cells of adipose origin, 50 per cent, Hyaluronic Acid at 30 per cent and TGF-beta, i.e. transforming growth factor beta, at 20 per cent.

## Description

### Field of the invention.

Subject of this invention is a support structure composed of a biomaterial with biodegradable components, with the function of support structure and the function of cultural medium for the second part, i.e. that composed of cells, which by amalgamating and inhibiting the support structure consisting of the first part replaces the biodegradable material with cellular biological material. The resulting material is therefore biodegradable in situ, with growth of the cells layer through appropriate external stimulation.

### State of the art.

Currently, arthritic forms with cartilage loss are treated by applying protheses or with injections of lubricating and moisturising substances, such as hyaluronic acid or similar. Prosthetic surgeries can be complex and difficult to carry out, while in situ injection of a hyaluronic acid have a temporary effect on joint contacts by acting as a shock absorber and lubricant within the joints, but unfortunately limited in time. Hydrogels with substances that reduce friction between the joints are also used, but, being hydrogel they have a hight water content and are not efficient in people with an above-overrange weight, i.e. in the overweight and obese range, which make up a large proportion of the patients requiring this type of intervention. Documents US 2013/084636 A1 and WO 2014/143925 A1 are known, in which stem cells are placed within a support structure, but the growth of these cells cannot be stimulated from outside and is not implemented after the layer forming the support structure is absorbed or degraded.

### Exposition of the invention.

The invention thus avoids the aforementioned disadvantages by introducing biocompatible, i.e. non-toxic substances into the body that promote cell growth in the parts where they are placed. The invention then has a rubber consistency when thickened and can withstand loads and mechanical stress, thus suitable for use by overweight or obese people. The rubber consistency then allows it to be both injected and suitable for printing using 3D printers. Stem cells are also stimulated in their growth by ultrasound, with external ultrasound stimulation acting on a component introduced into structure. In a preferred but not exclusive form of realisation the stem cell support structure comprises a 60 per cent biomaterial, consisting of 75 per cent Silicone by weight, Poli Glycolic Acid, i.e. PGA at 1.87 per cent, Xanthan gum at 1.25 per cent, NaCl, i.e. salt, at 3.75 per cent, Agar at 3.75 per cent, Carbon fullerene C60 at 4.38 per cent and water al 2.5 per cent; all reacted with 7.5 per cent Silicone catalyst. The remaining 40 per cent is predominantly cellular materials composed of ASC, i.e. mesenchymal cells of adipose origin, 50 per cent, hyaluronic acid 30 per cent and TGF-beta, i.e. transforming growth factor beta, 20 per cent. The biomaterial and cellular material are then placed in a syringe, shaken to amalgamate the biomaterial with the predominantly cellular part and then injected into the join in the case of cartilaginous prostheses. The invention has many proprieties, including biocompatibility, being resistant to many load stresses, and having carbon in its composition, the material is piezoelectric, and through external ultrasonic stimulation, allows the cellular material to be stimulated, regenerating it so much an extent that it absorbs the biomaterial and goes on to recompose the human biological part where it was worn out or absent and for which reconstruction was necessary. The invention, in another form of application, is placed in a 3D printer in the form of filament for FDM printers, i.e. for fused modelling printers. In this case, high temperatures are not required for moulding, as the material reacts at the room temperature. As a guide parameter for FDM printers, maximum temperatures of forty degrees centigrade for the printer bed and extruder, with fast drying. In a further form of application for 3D injection printers the invention must be introduced into the housing for liquid moulding. The invention has application in various fields: in orthopaedics for cartilage prostheses in all parts of the body. In cardiology for the fabrication of aortic prostheses and realisation of the heart muscle in 3D, with cells subsequently placed in the muscle implanted with engineered cells( using CRISPR genetic editing mode and subsequently stored by heart impulse) for the functioning of the heartbeat , in cases of unborn children diagnosed with congenital cardiomyopathy and/or malformations of the heart muscle; in internal medicine, in the reconstruction through 3D printing and subsequent implantation of cells of certain parts of the human body, including, for example the colon and oesophageal canal, as soft parts that falls within the scope of application of the invention; in cosmetic surgery for any type of prosthesis, such as breast and nose implants. It is also used in veterinary medicine for the reconstruction of worn animal parts, such as horse joints. The invention finds industrial application in the supply of the substances indicated for the support and for parts made from the same substances. Allthous some form of fabrication have been described, numerous modifications and variations are possible to experts in the field or as field evolves. These changes are therefore to be understood as being included in this description and in the claims. The invention is therefore susceptible to numerous modifications and variations all falling within the scope of the inventive concept and sanctioned by the following claims.

## Claims

1. Scaffold with stem cells **characterized in** comprising 60% biomaterial consisting, as weight percentage, 75% silicone, 1,87% polyglycolic acid, i.e. PGA, 1,25% xanthan gum, 3,75% NaCl, i.e. salt, 3,75% agar, 4,38% fullerene carbon C60 and 2,5% water, all reacted with 7,5% catalyst for silicone and 40% mainly cellular material consisting 50% ASC, i.e. mesenchymal cells of adipose origin, 30% hyaluronic acid and 20% TGF-beta, i.e. transforming growth factor beta.

2. Scaffold with stem cells, according to previous claim, **characterized in that** the biomaterial and the mainly cellular material are placed into a syringe, shaken to mix the biomaterial with the mainly cellular material and then injected into the joint.

3. Scaffold with stem cells, according to claim 1, **characterized in that** the biomaterial and the mainly cellular material are placed in a 3D printer in filament form.

4. Scaffold with stem cells, according to claims 1 and 3, **characterized in that** the 3D printer is a FDM printer, i.e. a fusion deposition modelling printer.

5. Scaffold with stem cells, according to claim 1, **characterized in that** the biomaterial and the mainly cellular material are placed in 3D injection printer.

6. Scaffold with stem cells, according to claims 1, 3, 4 or 5, characterized is used in orthopedics for cartilage prosthesis.

7. Scaffold with stem cells, according to claims 1, 3, 4 or 5, **characterized in that** is used in cardiology for aortic prosthesis and realization of heart muscle in 3D.

8. Scaffold with stem cells, according to claims 1, 3, 4 or 5, **characterized in that** is used in the internal medicine in the reconstruction of human body parts, such as the colon and the esophageal canal.

9. Scaffold with stem cells, according to claims 1, 3, 4 or 5, **characterized in that** is uses in cosmetic medicine for the realization of any kind of prosthesis, such as breast and nose implants.

10. Scaffold with stem cells, according to claims 1, 3, 4 or 5, **characterized in** is used in veterinary for the reconstruction of worn parts of the animal, such as horse joints..
